# EUROPEAN PATENT APPLICATION

(11) **EP 4 624 915 A1**
(43) Date of publication of application: **01.10.2025**
(21) Application number: 23894056.3
(22) Date of filing: 23.11.2023
(51) Int. Cl.: G01N 27/49, G01N 33/14, C07C 321/04, H01M 4/04

(54) **SENSOR FOR DETECTING MERCAPTANS IN WINES AND METHOD FOR DETERMINING SAME**

(30) Priority: 24.11.2022 ES 202231013
(71) Applicant: Universidad De Burgos, 09001 Burgos (ES)
(72) Inventor: PORTUGAL GÓMEZ, Paula, 09001 Burgos Burgos (ES); DOMÍNGUEZ RENEDO, Olga, 09001 Burgos Burgos (ES); ALONSO LOMILLO, M. Asunción, 09001 Burgos Burgos (ES)
(74) Representative: Capitán García, Maria Nuria
(86) International application number: PCT/ES2023/070698
(87) International publication number: WO 2024/110685

(57) **Abstract**

The invention provides a sensor for the detection of mercaptans in wines, which consists of a screen-printed carbon electrode modified with cobalt phthalocyanine and is specifically designed to detect the organoleptic defect associated with the presence of mercaptans in wines. The sensor enables a simple detection method while avoiding direct contact with the samples to be analysed, thereby facilitating routine and in situ analyses. The purpose of this is to enable early preventive diagnoses at the lowest possible cost.

## Description

### TECHNICAL FIELD OF THE INVENTION

This invention falls within the field of electrochemistry applied to chemical analysis, particularly for the detection of mercaptans in wines.

This invention relates to a sensor for detecting mercaptans in wines and to the amperometric method used for detecting them with that sensor.

More specifically, the invention provides a sensor for detecting mercaptans in wines, consisting of a screen-printed carbon electrode modified with cobalt phthalocyanine. In addition, the invention provides a "headspace" method for the detection of mercaptans in wines using the aforementioned sensor.

Both the sensor and the "headspace" detection method of the invention make it possible to detect the organoleptic defect associated with the presence of mercaptans in wines in a simple way, avoiding direct contact between the sensor and the wine samples, thus facilitating routine and in situ analyses. This enables to carry out early-stage preventive diagnoses at a minimal cost.

### BACKGROUND OF THE INVENTION

In the agri-food industry in general, and in the field of oenology in particular, there is a growing interest in the production of high-quality products. The constant quest for innovation in production processes is vital in an industry that aims to consolidate its excellent international reputation and to further improve its prospects. The wine sector is strategic for the European Union as it boasts major producing countries such as Italy, France, and Spain. According to data from the Ministry of Agriculture, the value of wine production in 2013 amounted to 2.143 billion euros, with half of the production destined for export, generating a positive trade balance of approximately 2.6 billion euros for the sector. Vineyard cultivation labour generates nearly 18 million workdays, making this a sector of extraordinary relevance not only for its economic impact but also for its social and environmental significance, as well as for the role of wine as an ambassador of national identity abroad.

The current wine market demands products with a harmonious composition, free from organoleptic defects, while preserving their taste and their desirable, fruity and varietal aromas. Physical and chemical reactions, as well as the absorption of foreign substances, can lead to wine faults such as sulphurous notes, metallic defects, or off-flavours like tannins or cork taint. Generally, these defects can be reduced or even eliminated within certain limits by applying appropriate treatments. Therefore, the prompt and accurate identification of such defects is highly useful in correcting the issue before it becomes significant and irreversible, potentially damaging the reputation and financial standing of the winery. It is therefore crucial that thorough quality control tests are carried out at all the stages, i.e. from the health status of the grapes, through the winemaking process, to the final product, in order to obtain defect-free wines.

It is generally possible to associate organoleptic deviations in wine with the specific group of chemical substances responsible, which enables their objective identification.

Sulphur compounds, which are primarily formed during the fermentation processes, also contribute to the characteristic aroma of each wine. However, when some of these compounds are produced in higher - than - normal amounts, they result in alterations in both the aroma and the flavour. For instance, hydrogen sulphide (H₂S), which is essential during the fermentation stage, can react with ethanol or acetaldehyde to produce mercaptans that lead to olfactory defects [C. Flanzy, Enologia: fundamentos cientificos y tecnológicos, 2nd ed., A. Madrid Vicente: Mundi-Prensa, Madrid, 2003; A. Belancic Majcenovic, et al., Synthesis and Stable Isotope Dilution Assay of Ethanethiol and Diethyl Disulfide in Wine Using Solid Phase Microextraction. Effect of Aging on Their Levels in Wine, J. Agric. Food Chem. 50 (2002) 6653-6658]. In addition, dimethyl sulphide contributes to the bouquet of mature wines and to the aroma of late harvest wines, but it can also be reduced to mercaptans by yeasts. During fermentation, esters of thioacetic acid are also formed. These have higher olfactory thresholds (200-300 µg/l) than their typical concentration in wine (2-20 µg/l). Through hydrolysis, however, they produce mercaptans with much lower sensory thresholds. The formation of trace amounts of these compounds is considered to be only a minor anomaly in the biochemistry of fermentation, although their sensory impact is significant and detrimental, as they can alter the aroma of bottled wines. The range of associated odours includes rotten eggs, burnt rubber, cooked asparagus, garlic, onion, and cabbage. This defect is well known among winemakers and wine consumers. Owing to the increasing sensitivity of consumers, abnormal odours related to sulphur compounds are the most common cause of complaints.

Therefore, their detection is of great interest in enabling early preventive diagnoses at a minimal cost.

The analysis of mercaptans has generally been addressed using chromatographic techniques, typically preceded by various preconcentration or separation steps due to the low concentration of these compounds in wine samples. Such steps include solid-phase extraction [A. Belancic Majcenovic, et al., Synthesis and Stable Isotope Dilution Assay of Ethanethiol and Diethyl Disulfide in Wine Using Solid Phase Microextraction. Effect of Aging on Their Levels in Wine, J. Agric. Food Chem. 50 (2002) 6653-6658; L. Mateo-Vivaracho, et al., Selective preconcentration of volatile mercaptans in small SPE cartridges: Quantitative determination of trace odor-active polyfunctional mercaptans in wine, J. Sep. Sci. 32 (2009) 3845-3853], headspace solid-phase microextraction [Y. Fang, et al., Sensitive quantification of sulfur compounds in wine by headspace solid-phase microextraction technique, J. Chromatogr. A. 1080 (2005) 177-185], liquid-liquid extraction [C. Pizarro, et al., Development of a dispersive liquid-liquid microextraction method for the simultaneous determination of the main compounds causing cork taint and Brett character in wines using gas chromatography-tandem mass spectrometry, J. Chromatogr. A. 1218 (2011) 1576-1584], or stir bar sorptive extraction [C. Herrera, et al., Development of a stir bar sorptive extraction method for the determination of volatile compounds in orange juices, J. Sep. Sci. 39 (2016) 3586-3593].

Electrochemical techniques, which also offer excellent detection capabilities, are more versatile for detecting this type of compounds, which can undergo electrochemical oxidisation or reduction at the working electrode available, account being taken of the significantly lower cost and the compact size of the electrochemical equipment, making it ideal for in situ analyses. Nevertheless, these techniques have not been widely used for the determination of mercaptans in wine.

To date, they have only been employed for the determination of ethanethiol in wines as a representative mercaptan. These include the use of hanging mercury drop electrodes in the concentration range of 200 to 600 µg/l [A. Guarda, et al., Simultaneous Determination of Ethanethiol, Inorganic Sulphide, and Sulphite in Wines by Cathodic Stripping Voltammetry, Food Anal. Methods. 10 (2017) 837-844], more environmentally friendly alternatives such as glassy carbon electrodes modified with gold nanoparticles (7.7 to 36.6 µg/l), screen-printed carbon electrodes (SPCEs) modified with gold nanoparticles (concentration range from 1.6 to 10.9 µg/l) [M.A. Alonso-Lomillo, et al., Electrochemical Detection of Mercaptans in Wine Using Gold Nanoparticle-Modified Carbon Electrodes, J. Electrochem. Soc. 168 (2021)], and glassy carbon electrodes modified with molecularly imprinted polymers (concentration range from 300 to 3100 µg/l) [O. Dominguez-Renedo, et al., Molecularly imprinted polypyrrole based electrochemical sensor for selective determination of ethanethiol, Talanta 253 (2023) 123936].

In this complex matrix, not only are mercaptans key redox-active species, but so are phenols, sulfites, ascorbic acid, glutathione, ethanol, oxygen, quinones, metal ions, and others [P.A. Kilmartin, Electrochemistry applied to the analysis of wine: A mini-review, Electrochem. Commun. 67 (2016) 39-42]. In this context, the use of sensors based on cobalt phthalocyanine (CoPh) would be advantageous as this electrocatalytic material allows the monitoring of thiols at significantly low potentials [S. Griveau, M. Gulppi, J. Pavez, J.H. Zagal, F. Bedioui, Cobalt phthalocyanine-based molecular materials for the electrocatalysis and electroanalysis of 2-mercaptoethanol, 2-mercaptoethanesulfonic acid, reduced glutathione and L-cysteine, Electroanalysis. 15 (2003) 779-785].

### DESCRIPTION OF THE INVENTION

This invention is set and defined in independent claims, while the dependent claims describe additional features thereof.

According to a first aspect, the purpose of this invention is to provide a sensor for the detection of mercaptans in wines, which consists of a screen-printed carbon electrode modified with cobalt phthalocyanine, wherein the redox mediator cobalt phthalocyanine is made up of 10µL of a 4.8 % (w/v) solution in ethanol. This sensor is specifically designed to detect the organoleptic defect associated with the presence of mercaptans in wine, in a simple way and avoiding direct contact between the sensor and the wine samples to be analysed. This facilitates the completion of routine and in situ analyses, enabling early preventive diagnoses at a minimal cost.

As per a second aspect, the invention provides a headspace-type amperometric method for the detection of mercaptans in wines using the aforementioned sensor. This approach avoids direct contact between the electrochemical devices and the sample, which may undoubtedly be very advantageous in ongoing monitoring testing during the various stages of the winemaking process, as it ensures that the product remains unaltered. Moreover, the number of interfering species is significantly reduced, since only volatile species are capable of influencing the analytical signal.

### DETAILED DESCRIPTION OF THE INVENTION

First, this invention provides a sensor for the detection of mercaptans in wines, which consists of a screen-printed carbon electrode system composed of a working electrode, an auxiliary or counter - electrode, both being carbon electrods, and a silver reference electrode, where the active surface of the working electrode is modified with the redox mediator cobalt phthalocyanine .

The screen-printed carbon electrodes, which are made up of a working electrode and an auxiliary or counter-electrode, both being carbon electrodes, and a silver/silver chloride reference electrode, are not particularly limited; for example, the DRP-C11L screen-printed electrodes supplied by Dropsens^{®} (Metrohm DropSens, Oviedo, Spain) or similar devices may be used.

The working surface is further modified with a cobalt phthalocyanine (II) (CoPh) solution in ethanol, specifically 10 µL of a 4.8 % (w/v) CoPh solution in ethanol, which is drop-casted onto the surface and left to dry at room temperature.

Furthermore, the invention also provides a headspace-type method for the amperometric detection of mercaptans in wines using the above-described sensor.

The method therefore includes the following steps:
i. Pour 1 mL of Britton-Robinson buffer at pH 2.6, containing 0.1 M KCl, as a supporting electrolyte, into a sealed cell;
ii. Wet the aforementioned detection sensor in the supporting electrolyte, place it in the upper part of the sealed cell, above the solution, and connect it to a potentiostat;
iii. Apply a constant potential of +0.8 V under stirring conditions until a stable current is registered;
iv. Introduce a volume of the wine sample to be analysed into the sealed cell;
v. Record the increase in current resulting from the oxidisation process occurring at the working electrode placed in the headspace of the cell, as per the following:

   *2Co*²⁺ + *RSH* ↔ *2Co*⁺ + *RSSR + 2H*⁺

   *2Co⁺* ↔ *2Co²⁺* + *2e⁻*
vi. Correlate the current recorded at the working surface of the sensor with the concentration of mercaptans in the cell.

The invention is further described below with reference to the following examples and figures.

In the figures:

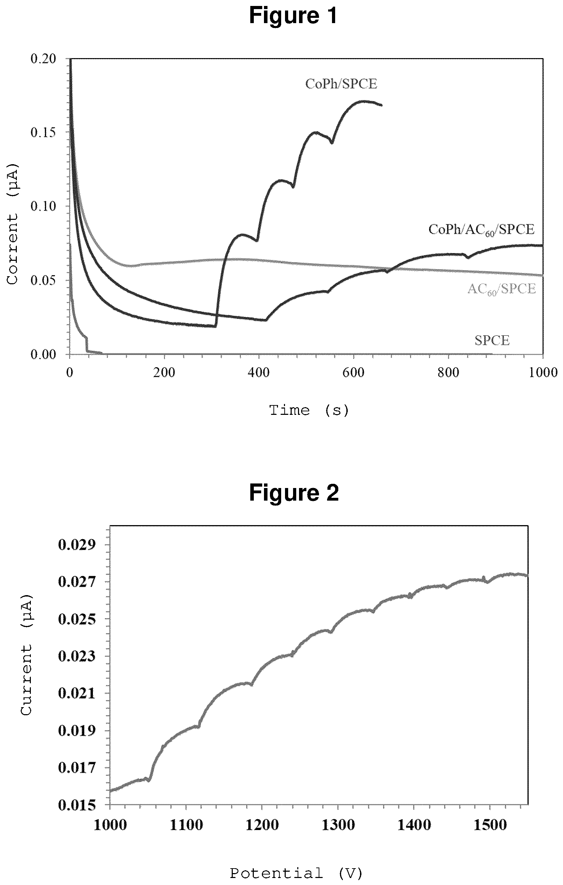

Figure 1: Amperograms recorded at +0.5 V using a screen-printed carbon electrode (SPCE), a screen-printed carbon electrode modified with cobalt phthalocyanine (CoPh/SPCE), a screen-printed carbon electrode modified with fullerene C₆₀ (AC₆₀/SPCE), and a screen-printed carbon electrode modified with both cobalt phthalocyanine and fullerene C₆₀ (CoPh/AC₆₀/SPCE) for successive additions of 50 µL of a 100 mg/L ethanethiol solution (representing mercaptans) in 1 mL of supporting electrolyte at pH 4.
Figure 2: Amperogram recorded at +0.8 V using a screen-printed carbon electrode modified with cobalt phthalocyanine (CoPh/SPCE) for successive additions of 10 µL of a 1 mg/L ethanethiol solution (representing mercaptans) in 1 mL of supporting electrolyte at pH 2.6.

### Examples

In the following examples, all the reagents used were of analytical reagent grade. All the solutions were prepared with Milli-Q water (Millipore, Bedford, MA, USA). 0.1 M KCl (Merck, Darmstadt, Germany) and Britton-Robinson (BR) buffer solutions - containing 0.04 M phosphoric acid (Panreac, Barcelona, Spain), 0.04 M acetic acid (VWR Chemical, Fontenay, France), and 0.04 M boric acid (Panreac, Barcelona, Spain) - were used as a supporting electrolyte for electrochemical measurements. 1 M NaOH solutions (Ecros, Barcelona, Spain) were employed to adjust the pH value of the buffer solutions.

Cobalt phthalocyanine (CoPh) solutions were prepared by dissolving cobalt phthalocyanine (II) (Alfa Aesar, Karlsruhe, Germany) in ethanol (VWR Chemicals, Rosny-sous-Bois, France) as solvent. Ethanethiol (EtSH) solutions, representing mercaptans, were prepared by dissolving the appropriate amount in Milli-Q water.

Screen-printed carbon electrodes (SPCE) (DRP-C11L, DRP-110CNT, DRP-110GPHOX, DRP-110RGPHOX, 110CSQD) supplied by Dropsens (Metrohm DropSens, Oviedo, Spain) were used for mercaptan electroanalysis.

Electrochemical measurements were performed using a PalmSens4 potentiostat (Palmsens BV, Houten, the Netherlands), using SPCEs with different modifications on the working electrode surface.

### Example 1: Modification of the working electrode with cobalt phthalocyanine

The modification of the working electrode of various SPCEs with CoPh was carried out via drop-casting. A volume of 10 µL of a 4.8 % (w/v) CoPh solution in ethanol was deposited onto the electrode surface - except during the optimization process - and left to dry at room temperature.

### Example 2: Electrochemical Measurements

Before using the devices, the screen-printed carbon electrodes (SPCEs) were wetted with the appropriate supporting electrolyte, so that they became pre-loaded by adsorption. They were then placed in the headspace of a sealed cell (Metrohm DropSens, Oviedo, Spain), containing 1 mL of Britton-Robinson (BR) buffer at pH 2.6, and amperometric measurements were performed by applying a constant potential of +0.8 V (except during the optimization process), under stirring conditions. Once a steady current was reached, a given volume of the corresponding analyte solution was added to the cell, and the corresponding increase in the current due to the oxidisation process occurring at the electrode surface was recorded.

First, the electrocatalytic responses of cobalt phthalocyanine (CoPh) were studied and compared with bare working electrodes for the amperometric detection of ethanethiol (EtSH), using different types of SPCEs: carbon (DRP-C11L), multi-walled carbon nanotubes functionalized with carboxyl groups (MWCNT-COOH, DRP-110CNT), graphene oxide (DRP-110GPHOX), reduced graphene oxide (DRP-110RGPHOX), and quantum dot-based electrodes ZnS/CdSe (DRP-110CSQD). In most of the registered amperograms, a noisy current of little analytical value was found, except when the carbon working electrode was modified with CoPh (CoPh/SPCE) and with CoPh and fullerene C60 (CoPh/AC60/SPCE). Figure 1 shows that the oxidisation current is significantly enhanced when using CoPh/SPCE, so it was decided to continue working with this device to quantify mercaptans.

Selectivity in amperometry is related to the redox potential of the analyte of interest. In addition to the applied potential, the pH of the supporting electrolyte and the concentration of the CoPh solution used to modify the electrode (expressed as a percentage) are experimental variables that greatly influence the oxidisation current registered as an analytical response. Therefore, an experimental mathematical model design was carried out to determine the relationship between these variables and the oxidisation current of an EtSH solution (98 µg/L), taken as the analytical response. The applied conditions are summarized in Table 1 as follows:

**Table 1**

| | pH of supporting electrolyte | Concentration of CoPh solution (%) | Potential applied (V) |
|---|---|---|---|
| 1 | 4 | 15 | 0.3 |
| 2 | 8 | 15 | 0.3 |
| 3 | 4 | 45 | 0.3 |
| 4 | 8 | 45 | 0.3 |
| 5 | 4 | 15 | 0.7 |
| 6 | 8 | 15 | 0.7 |
| 7 | 4 | 45 | 0.7 |
| 8 | 8 | 45 | 0.7 |
| 9 | 2.6 | 30 | 0.5 |
| 10 | 9.4 | 30 | 0.5 |
| 11 | 6 | 4.8 | 0.5 |
| 12 | 6 | 55.2 | 0.5 |
| 13 | 6 | 30 | 0.2 |
| 14 | 6 | 30 | 0.8 |
| 15 | 6 | 30 | 0.5 |
| 16 | 6 | 30 | 0.5 |
| 17 | 6 | 30 | 0.5 |

The results were evaluated using the Statgraphics software (STATGRAPHICS, STATGRAPHICS Centurion 19, Statgraphics Technol. Inc.), obtaining the following regression equation for the fitted model: Oxidisation current of EtSH = -2.63662 - 0.572209 × pH - 0.269559 × CoPh concentration + 37.3274 × Applied potential + 0.0709905 × pH2 + 0.026476 × pH × CoPh concentration - 2.75445 × pH × Applied potential + 0.00390899 × CoPh2 concentration - 0.266385 × CoPh concentration × Applied potential - 8.94781 × Applied potential2.

Using these values, the combination that maximized the oxidisation current of EtSH within the indicated region was as follows:
Supporting electrolyte pH = 2.6, applied potential = +0.8 V, and CoPh solution concentration = 4.8 % (w/v).

Under these experimental conditions, a well-defined amperometric response due to EtSH oxidisation on the working electrode surface was observed (Figure 2).

The current response measured at the working electrode surface depends on the concentration of EtSH. Therefore, different calibration curves were constructed under the optimal conditions of the experimental variables within the EtSH concentration range of 9.9 to 82.6 µg/L to validate the analytical method in terms of precision and detection capability. Precision, which is generally expressed as a relative standard deviation (RSD), often varies with analyte concentration; hence, considering the slopes of such regressions avoids this drawback since different concentration levels are assessed simultaneously. An RSD of 7 % (n = 5) was obtained for reproducibility, highlighting the agreement between the results recorded using different CoPh/SPCE electrodes. Regarding detection capability (CCβ) and decision limit (CCα), values of 12.5 µg/L and 6.5 µg/L (α = β = 0.05) were found, respectively.

The selectivity in the determination of EtSH was also studied using the described procedure, taking into account the potential interference from other mercaptans, such as 4-mercaptobenzoic acid, or phenolic compounds, such as 4-ethylphenol or 4-ethylguaiacol. Under the optimal experimental variable conditions for EtSH detection, no alteration of the recorded amperometric signal was observed due to the presence of these compounds, even at concentrations exceeding 1 mg/L; thus, they were not considered to be interferents.

Finally, the applicability of the described method was validated through recovery experiments, in which wines were analysed both in their original state and after the addition of a known amount of analyte to the test portion, as per the guidelines of M. Thompson, S.L.R. Ellison, R. Wood, Harmonized guidelines for single-laboratory validation of methods of analysis (IUPAC Technical Report), Pure Appl. Chem. 74 (2002) 835-855.

Consequently, two white wine samples and two red wine samples were analysed by applying the standard analyte addition method to an aliquot of each wine sample, as per the following procedure:
i. Pour 1 mL of Britton-Robinson buffer at pH 2.6, containing 0.1 M KCl into a sealed cell, as a supporting electrolyte;
ii. Wet the aforementioned detection sensor in the supporting electrolyte, place it in the upper part of the sealed cell, above the solution, and connect it to a potentiostat;
iii. Apply a constant potential of +0.8 V under stirring conditions until a stable current is registered;
iv. Introduce a volume of the wine sample to be analysed into the sealed cell, registering an increase in current; once the current becomes stable again, introduce a volume of the EtSH stock solution into the cell;
v. Repeat the addition of volumes of the EtSH stock solution into the cell several times until no further current increases are observed;
vi. Correlate the current recorded at the working electrode surface of the sensor with the concentration of mercaptans present in the cell.

The results are shown in Table 2 below:

**Table 2**

| **Sample** | **Added concentration (ppb)** | **Measured concentration (ppb)** | **Recovery (%)** |
|---|---|---|---|
| **White Wine 1** | - | No detected | - |
| | 9.8 | 10.0 ± 0.2 | 102.4 % |
| | 19.2 | 19.3 ± 0.4 | 100.2 % |
| **White Wine 2** | - | No detected | - |
| | 9.8 | 9.6 ± 0.2 | 97.9 % |
| | 19.2 | 19.5 ± 0.3 | 101.4 % |
| **Red Wine 1** | - | No detected | - |
| | 9.8 | 10.1 ± 1.0 | 103.1 % |
| | 19.2 | 19.4 ± 0.5 | 100.9 % |
| **Red Wine 2** | - | No detected | - |
| | 9.8 | 9.5 ± 0.8 | 96.8 % |
| | 19.2 | 19.7 ± 2.9 | 102.5 % |

As shown in the above table, no presence of EtSH was detected in any of the samples. Subsequently, recovery experiments were performed by analysing two levels of spiked wine samples, obtaining good recovery values ranging between 97 % and 103 %.

### Comparative Examples:

The described method clearly outperforms the previously reported methods for the detection of EtSH in wine samples. In terms of concentration range, 9.9 to 82.6 µg/L, the described method is comparable to the best methods previously used for this purpose, with the advantage that, by measuring in the headspace, interferences are reduced and, most importantly, the device does not come into direct contact with the wine sample, thus avoiding alteration of the product.

In this regard, for the detection of mercaptans in wine using a mercury electrode, the concentration values range from 200 to 600 µg/L [A. Guarda, et al., Simultaneous Determination of Ethanethiol, Inorganic Sulfide, and Sulfite in Wines by Cathodic Stripping Voltammetry, Food Anal. Methods. 10 (2017) 837-844]. For the detection of mercaptans in wine using a glassy carbon electrode modified with AuNPs, the concentration values range from 7.7 to 36.6 µg/l [M.A. Alonso-Lomillo, et al., Electrochemical Detection of Mercaptans in Wine Using Gold Nanoparticle-Modified Carbon Electrodes, J. Electrochem. Soc. 168 (2021)]. For the detection of mercaptans in wine using a screen-printed carbon electrode modified with AuNPs, the concentration values range from 1.6 to 10.9 µg/L [M.A. Alonso-Lomillo, et al., Electrochemical Detection of Mercaptans in Wine Using Gold Nanoparticle-Modified Carbon Electrodes, J. Electrochem. Soc. 168 (2021)]. Finally, for the detection of mercaptans in wine using a glassy carbon electrode modified with molecularly imprinted polymers and AuNPs, the concentration values range from 300 to 3100 µg/L [O. Dominguez-Renedo, et al., Molecularly imprinted polypyrrole based electrochemical sensor for selective determination of ethanethiol, Talanta 253 (2023) 123936].

## Claims

1. Sensor for the detection of mercaptans in wines, consisting of a screen-printed device comprising a carbon working electrode and a carbon auxiliary or counter-electrode, and a silver/silver chloride reference electrode, wherein the active surface of the working electrode is modified with 10 µL of a 4.8 % (w/v) solution of the redox mediator cobalt phthalocyanine in ethanol.

2. Headspace-type method for the amperometric detection of mercaptans in wines, comprising the following steps:
i. Pour 1 mL of Britton-Robinson buffer at pH 2.6, containing 0.1 M KCl into a sealed cell, as a supporting electrolyte;
ii. Pre-wetting the mercaptan detection sensor according to any of claims 1 to 2 in the supporting electrolyte, and connecting it to a potentiostat;
iii. Applying a constant potential of +0.8 V under stirring conditions until a steady current is recorded;
iv. Introducing a volume of the wine sample to be analysed into the sealed cell;
v. Recording the increase in current resulting from the oxidisation process which occurs at the working electrode of the mercaptan detection sensor according to any of claims 1 to 2 in the headspace of the cell;
vi. Correlating the current recorded at the working surface of the mercaptan detection sensor as per any of claims 1 to 2 with the concentration of mercaptans in the cell.
